Europäisches Patentamt

European Patent Office

Office européen des brevets

(19)

(11) **EP 1 248 758 B1**

(12) **EUROPÄISCHE PATENTSCHRIFT**

(45) Veröffentlichungstag und Bekanntmachung des Hinweises auf die Patenterteilung:
**30.06.2004 Patentblatt 2004/27**

(21) Anmeldenummer: **00972895.7**

(22) Anmeldetag: **08.11.2000**

(51) Int Cl.$^7$: **C07C 31/30**

(86) Internationale Anmeldenummer:
**PCT/EP2000/011027**

(87) Internationale Veröffentlichungsnummer:
**WO 2001/038277 (31.05.2001 Gazette 2001/22)**

(54) **KONZENTRIERTE, STABILE ALKALIALKOHOLATLÖSUNGEN**

CONCENTRATED, STABLE ALKALI ALCOXIDE SOLUTIONS

SOLUTIONS CONCENTREES D'ALCOOLATE ALCALINES STABLES

(84) Benannte Vertragsstaaten:
**AT BE CH CY DE DK ES FI FR GB GR IE IT LI LU MC NL PT SE TR**

(30) Priorität: **24.11.1999 DE 19956558**

(43) Veröffentlichungstag der Anmeldung:
**16.10.2002 Patentblatt 2002/42**

(73) Patentinhaber: **Chemetall GmbH**
**60487 Frankfurt am Main (DE)**

(72) Erfinder:
• **DAWIDOWSKI, Dirk**
**60439 Frankfurt am Main (DE)**
• **EMMEL, Ute**
**65929 Frankfurt am Main (DE)**
• **WEISS, Wilfried**
**61440 Oberursel (DE)**
• **WIETELMANN, Ulrich**
**61381 Friedrichsdorf (DE)**

(74) Vertreter: **Uppena, Franz, Dr. et al**
**Dynamit Nobel AG**
**Patente, Marken & Lizenzen**
**53839 Troisdorf (DE)**

(56) Entgegenhaltungen:
**DE-A- 3 238 963**

**Beschreibung**

Die Erfindung betrifft konzentrierte stabile Lösungen von Alkalialkoholaten sekundärer und tertiärer Alkohole, Verfahren zur deren Herstellung und deren Verwendung.

Alkalimetallalkoholate R-OM (R = Alkyl mit 3 bis 20 C-Atomen, M = Li, Na, K, Rb, Cs) sind hydrolyseempfindliche Verbindungen, die wegen ihrer basischen Eigenschaften in der organischen Synthese häufig Verwendung finden. So dienen sie als Kondensations-, Veresterungs- und Alkylierungsmittel und zur Einführung der Alkoxygruppe in andere Verbindungen (Williamson-Synthese).

Alkalialkoholate werden im allgemeinen durch Einwirkung von Alkalimetallen auf Alkohole gem.

$$R\text{-OH} + M \rightarrow R\text{-OM} + \tfrac{1}{2}\, H_2 \uparrow \tag{1}$$

dargestellt. Neben dem elementaren Metall (M = Li, Na, K, Rb, Cs) sind auch andere reaktive Metallverbindungen ("Metallierungsmittel"), wie Alkalimetallhydride, -amide und Organoalkalimetallverbindungen (z.B. Butyllithium) verwendbar.

Die Umsetzung erfolgt zumeist in flüssiger Phase, d.h. in Gegenwart eines Lösungsmittels. Letzteres ist bei den Alkoholaten der primären Alkohole (z.B. Methanol, Ethanol, n-Butanol) vorzugsweise der primäre Alkohol selbst. Die Alkoholate sekundärer und tertiärer Alkohole sind in Alkoholen in der Regel nur schwer löslich. Deshalb werden sie häufig in aprotischen Lösungsmitteln (z.B. Kohlenwasserstoffe, Ether) hergestellt.

Die Alkoholate können nach Eindampfung der Syntheselösungen in Form fester (pulverförmiger) Produkte vermarktet werden. Der Nachteil dieser Handelsform besteht darin, daß Alkoholatstäube aufgrund ihrer Baseneigenschaften stark korrosiv wirken, d.h. es sind entsprechende Schutzmaßnahmen zu treffen, um den körperlichen Kontakt mit den Alkoholatpulvern auszuschließen. Um diesen handhabungsbedingten Nachteil zu vermeiden, sind flüssige Lieferformen, d.h. Lösungen besonders erwünscht. Lösungen sind jedoch ökonomisch nur dann von Interesse, wenn die Löslichkeit des Alkoholates über einen weiten Temperaturbereich gut ist, d.h. z.B. über 20 % liegt.

Die individuellen Löslichkeiten von Alkoholaten in einem Lösungsmittel hängen vor allem vom Metall M sowie dem Alkylrest ab. Im allgemeinen sind die vom Lithium abgeleiteten Alkoholate am besten löslich. Weiterhin ist die Löslichkeit eine Funktion der Raumfüllung und Sperrigkeit der Alkylgruppe: je "größer" diese ist, desto besser ist die Löslichkeit vorzugsweise in wenig polaren Lösungsmitteln (z.B. Kohlenwasserstoffen).

Neben diesen vom jeweiligen Alkohol und Metall geprägten Parametern gibt es jedoch noch weitere Einflußgrößen, die die individuelle Löslichkeit beeinflussen. Im Falle der Alkalialkoholate, die sich von sekundären und tertiären Alkoholen ableiten, ist dies der Restalkoholgehalt.

Es ist bekannt, daß Alkoholate mit freien Alkoholen Komplexe bilden, die in apolaren oder wenig polaren Lösungsmitteln schwerlöslich sind.

$$x\, R\text{-OM} + y\, R'\text{-OH} \rightarrow (R\text{-OM})_x \cdot (R'\text{-OH})_y \downarrow \tag{2}$$

Die Gegenwart von freiem Alkohol in Alkoholatlösungen ergibt sich entweder durch unvollständige Umsetzung gem. Gl. (1) oder ist eine Folge von Hydrolyse durch Luft- und/oder Wasserkontakt gem. Gl. (3):

$$R\text{-OM} + H_2O \rightarrow R\text{-OH} + MOH \tag{3}$$

Eigene Untersuchungen ergaben, daß z.B. im Falle von konzentrierten Natrium-tert-butylatlösungen in Methyl-tert-butylether (MTBE), Tetrahydrofuran (THF) oder Toluol die Stöchiometrie des gem. Gl. (2) gebildeten schwerlöslichen Komplexes x : y = ca. 3 bis 6 : 1 ist, d.h. freier Alkohol vermag eine deutlich über-stöchiometrische Menge an Alkoholat aus der Lösung zu entfernen.

Aus all dem leitet sich der Schluß ab, daß Lösungen der von sekundären und tertiären Alkoholen abgeleiteten Alkalialkoholate möglichst wasser- und alkoholfrei sein sollten, um maximale Alkoholatlöslichkeiten zu erreichen.

Die Aufgabe der vorliegenden Erfindung besteht darin, einen Weg aufzuzeigen, die Löslichkeit der von sekundären und tertiären Alkoholen abgeleiteten Alkalimetallalkoholate zu steigern und ihre Anfälligkeit gegenüber Hydrolyseeinflüssen zu verringern und so zur Stabilität dieser Alkalimetallalkoholatlösungen beizutragen.

Die Aufgabe wird durch die im Anspruch 1 angegebenen Lösungen gelöst, die Ansprüche 2 bis 8 geben Varianten der erfindungsgemäßen Lösungen an. Die Ansprüche 9 bis 12 geben Verfahren zur Herstellung der erfindungsgemäßen Lösungen an und der Anspruch 13 gibt eine Verwendung für die erfindungsgemäßen Lösungen an.

Der Erfindungsgedanke ist, daß ein Gemisch aus einem Alkoholat ROM und einem Alkalimetallhydroxid M'OH in einem aprotischen Lösungsmittel gelöst wird, wobei R = ein sekundärer oder tertiärer Alkylrest mit 3 bis 20 C-Atomen ist und wobei M und M' unabhängig voneinander = Li, Na, K, Rb oder Cs sind.

Sekundäre oder tertiäre Alkohole sind z.B. Isopropylalkohol, sec-Butylalkohol, tert-Butylalkohol oder tert-Amylalkohol.

Es wurde überraschend gefunden, daß schon geringe Mengen Alkalihydroxid ausreichen, um die Löslichkeit sekundärer und tertiärer Alkoholate wesentlich zu steigern. Dies wird am Beispiel des Systems Natrium-tert-amylat (NTA) / NaOH / Toluol demonstriert.

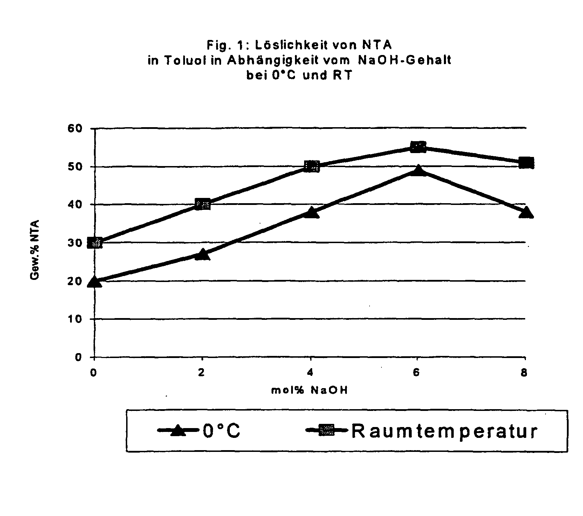

Wie Fig. 1 zu entnehmen ist, steigt die Löslichkeit von NTA in Toluol bei Raumtemperatur mit zunehmendem NaOH-Gehalt zunächst stark an, um nach Erreichen eines Maximums bei ca. 6 mol % schließlich wieder abzufallen.

Es wurde gefunden, daß ein Hydroxidgehalt im Bereich zwischen min. 0,5 und max. 15 Mol% (bevorzugte Bereiche sind in den Ansprüchen offenbart) ausreicht, um einen deutlich günstigen Effekt auf die Löslichkeit der Alkalialkoholate auszuüben. Für Anwendungen in der organischen Synthese ist ein solcher Hydroxidgehalt im allgemeinen nicht störend, da Alkalihydroxide weniger basisch als Alkoholate sind, d.h. es sind in der Regel keine unterschiedlichen Reaktionsergebnisse zu erwarten.

Um Ausfällungen schwerlöslicher Alkohol/Alkoholat-Komplexe zu vermeiden, sollte der Restalkoholgehalt möglichst gering sein. Der max. Restgehalt der Lösungen an freiem Alkohol beträgt max. 1,0 Mol%, bevorzugt max. 0,5 Mol%.

Als Lösungsmittel können polare und/oder unpolare aprotische Lösungsmittel verwendet werden. Eingesetzt werden können aromatische Kohlenwasserstoffe (z.B. Toluol, Benzol, Xylol, Ethylbenzol), oder offenkettige oder cyclische aliphatische Kohlenwasserstoffe (z.B. Pentan, Hexan, Cyclohexan, Heptan, Octan) oder Ether (offenkettige oder cyclische, ein- oder mehrfunktionelle, z.B. Diethylether, Tetrahydrofuran, 2Methyl-Tetrahydrofuran, Methyl-tert-butylether, 1,2-Dimethoxyethan (1,2-DME), Diethylenglykoldimethylether) oder Amide (z.B. Dimethylformamid DMF) oder Dimethylsulfoxid DMSO oder Acetale (z.B. Diethoxymethan oder Diethoxyethan) oder Nitrile (z.B. Acetonitril). Es können auch Mischungen dieser Stoffe verwendet werden.

Die Zumischung des Alkalimetallhydroxids kann auf unterschiedliche Art und Weise erfolgen:

Isolierte, pulverförmige Alkoholate können mit einem Alkalimetallhydroxid versetzt und vermischt und anschließend in einem wasserfreien, aprotischen Lösungsmittel aufgelöst werden. Falls das Hydroxid nicht in feinverteilter Form vorliegen sollte, müßte die Mischung gemahlen werden. Da die kommerziell verfügbaren Hydroxide nicht völlig wasserfrei sind (sie sind ausgesprochen hygroskopisch) läßt sich oftmals nicht vermeiden, daß auf diese Weise geringe Mengen Wasser eingeschleppt werden, die sich negativ auf die Löslichkeit auswirken. Dem kann durch Nachtrocknung der Lösung (Zugabe von M', M'H, Molekularsieb, azeotrope Wasserentfernung) begegnet werden.

Einfacher und wirkungsvoller ist es, die Hydroxidbeimischung bei der Alkoholatsynthese vorzunehmen. Dazu wird z.B. die berechnete Menge Hydroxid (M'OH) oder Wasser zum im Lösungsmittel suspendierten Metallierungsmittel (das ist das Alkalimetall selbst oder das Hydrid, Amid oder eine Organoalkalimetallverbindung) zudosiert. Als Lösungsmittel dienen die o.a. polaren und/oder unpolaren aprotischen Lösungsmittel. Eine besonders elegante Variante besteht auch darin, statt völlig wasserfreiem Alkohol eine im berechneten Verhältnis hergestellte Wasser/Alkohol-Mischung einzusetzen. Bei der Verwendung von Wasser für die in-situ-Herstellung von Alkalimetallhydroxid muß eine entsprechende Mehrmenge an Metallierungsmittel vorgelegt werden.

Dem Fachmann sind die verschiedenen verfahrenstechnischen Varianten bei der Alkoholatsynthese bekannt. So kann auch der Alkohol vorgelegt und das Metallierungsmittel zudosiert werden. Es gibt viele denkbare Varianten, wie und wann das Wasser bzw. das Metallhydroxid zugemischt werden.

Entscheidend in allen Fällen ist, daß sowohl der Alkohol als auch das Wasser möglichst quantitativ mit dem Metallierungsmittel abreagieren. Deshalb sollte das Metallierungsmittel je nach Verfahrensvariante vorzugsweise im Überschuß (ca. 1 bis 50 %, bevorzugt 1 bis 10 %) eingesetzt werden.

Es wurde gefunden, daß die Gegenwart geringer Wassermengen (0,5 bis 10 Mol%) im allgemeinen keinen signifikanten Einfluß auf den Ablauf der Bildungsreaktion (d.h. Reaktionsgeschwindigkeit, Selektivität etc.) ausübt. Es müssen deshalb keine weiteren vom Stand der Technik abweichende Maßnahmen ergriffen werden.

Verwendung finden die erfindungsgemäßen Alkoholatlösungen als Reagenzien für die organische Synthese.

Der Vorteil der erfindungsgemäßen sekundären und tertiären Alkoholatlösungen ist die gegenüber dem Stand der Technik höhere Konzentration der Lösungen bei gleichzeitiger Stabilität der Lösungen, was eine verfahrenstechnische und ökonomische Verbesserung darstellt.

Der Gegenstand der Erfindung wird anhand der folgenden Beispiele näher erläutert:

Beispiel 1: Löslichkeiten von MO$^t$Bu

Es wurden die Löslichkeiten von Kalium- und Natrium-tert-butylat (MO$^t$Bu mit M = K, Na) in Toluol oder Methyl-tert-butylether (MTBE) bei Zugabe von LiOH oder NaOH (M'OH mit M' = Li, Na) untersucht. Dazu wurde zunächst die Löslichkeit eines völlig Restbase-freien Alkoholates im Lösungsmittel bei einer bestimmten Temperatur bestimmt (mehrstündiges Rühren über einem Bodensatz ungelösten Alkoholates und Bestimmung der Alkalität sowie des Restbasegehaltes nach Karl Fischer). Dann wurde eine bestimmte Menge eines wasserfreien, pulverisierten Alkalihydroxids zugegeben und wieder einige Stunden bis zur Gleichgewichtseinstellung gerührt. Die Ergebnisse sind in Tabelle 1 aufgeführt.

Tab. 1:

| Löslichkeiten von MO$^t$Bu | | | | | |
|---|---|---|---|---|---|
| Alkoholat | Alkalihydroxid | | Lösungsmittel | Temperatur | Löslichkeit |
| | M'OH | Konz. (mol%) | | (°C) | (Gew.%) |
| KO$^t$Bu | - | - | Toluol | 0 | 2,9 |
| KO$^t$Bu | NaOH | 5 | " | 0 | 4,2 |
| KO$^t$Bu | LiOH | 5 | " | 0 | 3,6 |
| KO$^t$Bu | - | - | " | 25 | 3,4 |
| KO$^t$Bu | NaOH | 5 | " | 25 | 4,5 |
| NaO$^t$Bu | - | - | " | 0 | 3,8 |
| NaO$^t$Bu | NaOH | 3 | " | 0 | 4,0 |
| NaO$^t$Bu | NaOH | 5 | " | 0 | 4,8 |
| NaO$^t$Bu | NaOH | 1,6 | MTBE | 25 | 23,4 |
| NaO$^t$Bu | NaOH | 3,0 | " | 25 | 27,7 |

Aus Tabelle 1 ist ersichtlich, daß sich die Löslichkeit von Kalium-tert-butylat (KOtBu) in Toluol bei 0 °C durch Zusatz von 5 mol% LiOH um etwa 24 % und durch Zusatz von 5 mol% NaOH um etwa 45 % erhöhen lässt. Auch bei 25 °C ist ein positiver Effekt, wenn auch nicht so stark ausgeprägt, zu beobachten.

Ebenfalls wird die Löslichkeit von Natrium-tert-butylat sowohl in Toluol bei 0 °C als auch in MTBE bei Raumtemperatur durch Basenzusatz erhöht.

Vergleichsbeispiel A: Synthese von Na-tert-amoxid (NTA)-Lösung in Toluol (ohne M'OH-Zusatz)

In einem 2-l-Doppelmantelreaktor, ausgestattet mit Rückflußkühler und Tropftrichter wurden 103,3 g Natriumstücke (4,49 mol) in 600 g Toluol suspendiert und unter Rühren bis zur Siedetemperatur des Toluols erhitzt. Nach Aufschmelzen des Natriums wurde die Rührintensität verstärkt und 450 ml (4,09 mol) tert-Amylalkohol innerhalb 4 Stunden zugetropft.

Nach Beendigung der Zugabe wurde so lange weitergekocht, bis die Gasentwicklung vollständig aufhörte (5 Stunden).

Nach Abkühlung auf etwa 50 °C wurde die klare Produktlösung von den Natriumresten abdekantiert, in mehrere Schlenkkolben abgefüllt und bei verschiedenen Temperaturen einige Tage gelagert. Bestimmt wurde die Gesamtbase der Lösungen, woraus sich folgende Maximalgehalte an gelöstem Na-tert-amoxid (NTA-Gehalt) ergaben:

| Temperatur (°C) | Gesamtbase (mmol/g) | NTA-Gehalt (%) |
|---|---|---|
| 34 | 3,577 | 39,5 |
| 21 | 3,275 | 36,1 |
| 0 | 2,298 | 25,3 |

Beispiel 2: Synthese von NTA-Lösung in Toluol mit Na-Überschuß und 6 mol % H$_2$O-Zusatz

30 g Natrium (1,3 mol) wurden in 90 g Toluol eingetragen und am Kochpunkt mit einem Gemisch aus 82,9 g t-Amylalkohol (0,94 mol) und 1,08 g Wasser (60 mmol) umgesetzt. Nach einer dreistündigen Nachreaktionszeit verebbte die Gasentwicklung vollständig. Die dekantierte klare Lösung wurde in zwei Schlenkgefäße abgefüllt und bei 24 °C und 0 °C einige Tage gelagert. Bestimmt wurden die Gesamtbase und die freie Base der Lösungen, woraus sich folgende NTA-Gehalte ergaben:

| Temperatur (°C) | Gesamtbase (mmol/g) | freie Base[1] (mmol/g) | NTA-Gehalt (%) |
|---|---|---|---|
| 24 | 5,14 | 0,31 | 53,2 |
| 0 | 4,80 | 0,30 | 49,6 |

[1] Titration nach Karl Fischer

Beispiel 3: Synthese von NTA-Lösung in Toluol, Zusatz von 5 mol % NaOH

27,6 g (1,2 mol) Natriumstücke und 2,0 g (50 mmol) NaOH-Plätzchen wurden in 90 g Toluol suspendiert und bei Siedetemperatur mit 88,2 g t-Amylalkohol (1 mol) versetzt. Am Ende der zweistündigen Nachreaktionsphase waren noch Teile der NaOH-Plätzchen ungelöst.

Die dekantierte Lösung wurde bei zwei verschiedenen Temperaturen gelagert und es ergaben sich folgender NTA-Gehalte:

| Temperatur (°C) | Gesamtbase (mmol/g) | freie Base[1] (mmol/g) | NTA-Gehalt (%) |
|---|---|---|---|
| 23 | 4,40 | 0,165 | 46,6 |
| 0 | 3,94 | 0,185 | 41,3 |

[1] Titration nach Karl Fischer

Die Analyse ergab hier, daß nur etwa 70 % der eingesetzten NaOH-Menge in Lösung gegangen waren, d.h. der effektive NaOH-Zusatz betrug nur 3,5 mol %.

Die erfindungsgemäßen NTA-Lösungen aus den Beispielen 2 und 3 zeigen eine deutlich höhere NTA-Löslichkeit gegenüber der NTA-Lösung aus Vergleichsbeispiel A.

**Patentansprüche**

1. Lösungen von Alkalialkoholaten ROM, welche aus einem Alkalimetallkation und einem sekundären oder tertiären Alkoholrest bestehen, in aprotischen Lösungsmitteln, **dadurch gekennzeichnet, daß** die Lösungen bezogen auf den Alkoholatgehalt min. 1,0 und max. 15 Mol% Alkalimetallhydroxid M'OH und max. 1,0 Mol% freien Alkohol R-OH enthalten, wobei R = ein sekundärer oder tertiärer Alkylrest mit 3 bis 20 C-Atomen ist und wobei M und M' unabhängig voneinander = Li, Na, K, Rb oder Cs sind.

2. Lösungen nach Anspruch 1, **dadurch gekennzeichnet, daß** der Gehalt an Alkalimetallhydroxid M'OH min 1,5 Mol% beträgt.

3. Lösungen nach Anspruch 1 oder 2, **dadurch gekennzeichnet, daß** der Gehalt an Alkalimetallhydroxid M'OH max. 12 Mol% beträgt.

4. Lösungen nach Anspruch 1 oder 2, **dadurch gekennzeichnet, daß** der Gehalt an Alkalimetallhydroxid M'OH max. 10 Mol% beträgt.

5. Lösungen nach Anspruch 1 oder 2, **dadurch gekennzeichnet, daß** der Gehalt an Alkalimetallhydroxid M'OH max. 8 Mol% beträgt.

6. Lösungen nach einem der Ansprüche 1 bis 5, **dadurch gekennzeichnet, daß** die Lösungen max. 0,5 Mol% freien Alkohol R-OH enthalten.

7. Lösungen nach einem der Ansprüche 1 bis 6, **dadurch gekennzeichnet, daß** als aprotische Lösungsmittel Kohlenwasserstoffe (Aromaten oder offenkettige oder cyclische Aliphaten) oder Ether (offenkettige oder cyclische, ein- oder mehrfunktionelle) oder Amide oder Acetale oder Nitrile oder eine Mischung dieser Stoffe verwendet werden.

**8.** Lösungen nach einem der Ansprüche 1 bis 7, **dadurch gekennzeichnet daß** M = Na ist und R = tert-Amyl C$(CH_3)_2C_2H_3$ ist, das aprotische Lösungsmittel Toluol ist und die Konzentration von Na-tert-amoxid 41,3 bis 53,2 Gew.-% beträgt.

**9.** Lösungen nach einem der Ansprüche 1 bis 7, **dadurch gekennzeichnet, daß** M = Na ist und R = tert-Butyl C$(CH_3)_3$ ist, das aprotische Lösungsmittel Methyl-tert-Butylether ist und die Konzentration von Na-tert-butoxid 23,4 bis 27,7 Gew.-% beträgt.

**10.** Verfahren zur Herstellung von in den Ansprüchen 1 bis 9 angegebenen alkalimetallhydroxidhaltigen Alkoholatlösungen, **dadurch gekennzeichnet, daß** eine wasserfreie Mischung aus einem sekundären oder tertiären Alkalialkoholat ROM und Alkalimetallhydroxid M'OH in einem wasserfreien, aprotischen Lösungsmittel aufgelöst wird.

**11.** Verfahren zur Herstellung von in den Ansprüchen 1 bis 9 angegebenen alkalimetallhydroxidhaltigen Alkoholatlösungen, **dadurch gekennzeichnet, daß** eine Mischung aus einem sekundären oder tertiären Alkalialkoholat ROM und Alkalimetallhydroxid M'OH in einem aprotischen Lösungsmittel aufgelöst wird und die Lösung nachgetrocknet wird.

**12.** Verfahren zur Herstellung von in den Ansprüchen 1 bis 9 angegebenen alkalimetallhydroxidhaltigen Alkoholatlösungen, **dadurch gekennzeichnet, daß** ein Gemisch, bestehend aus Wasser und/oder Alkalimetallhydroxid, einem sekundären oder tertiären Alkohol und einem aprotischen Lösungsmittel, mit einem Metallierungsmittel umgesetzt wird, wobei als Metallierungsmittel ein Alkalimetall (Li, Na, K, Rb oder Cs) oder ein Alkalimetallhydrid oder ein Alkalimetallamid oder eine Organoalkalimetallverbindung eingesetzt wird.

**13.** Verfahren nach Anspruch 12, **dadurch gekennzeichnet, daß** als Organoalkalimetallverbindung Butyllithium eingesetzt wird.

**14.** Verwendung der in den Ansprüchen 1 bis 9 angegebenen Alkoholatlösungen als Reagenzien für die organische Synthese.

## Claims

**1.** Solutions of alkali alkoxides ROM, which consist of an alkali metal cation and a secondary or tertiary alcohol residue, in aprotic solvents, **characterised in that**, relative to alkoxide content, the solutions contain at least 1.0 and at most 15 mol% of alkali metal hydroxide M'OH and at most 1.0 mol% of free alcohol R-OH, wherein R is a secondary or tertiary alkyl residue having 3 to 20 C atoms and wherein M and M' are mutually independently Li, Na, K, Rb or Cs.

**2.** Solutions according to claim 1, **characterised in that** the content of alkali metal hydroxide M'OH is at least 1.5 mol%.

**3.** Solutions according to claim 1 or claim 2, **characterised in that** the content of alkali metal hydroxide M'OH is at most 12 mol%.

**4.** Solutions according to claim 1 or claim 2, **characterised in that** the content of alkali metal hydroxide M'OH is at most 10 mol%.

**5.** Solutions according to claim 1 or claim 2, **characterised in that** the content of alkali metal hydroxide M'OH is at most 8 mol%.

**6.** Solutions according to any one of claims 1 to 5, **characterised in that** the solutions contain at most 0.5 mol% of free alcohol R-OH.

**7.** Solutions according to any one of claims 1 to 6, **characterised in that** the aprotic solvents used are hydrocarbons (aromatics or open-chain or cyclic aliphatics) or ethers (open-chain or cyclic, mono- or polyfunctional) or amides or acetals or nitriles or a mixture of these substances.

**8.** Solutions according to any one of claims 1 to 7, **characterised in that** M is Na and R is tert-amyl C$(CH_3)_2C_2H_5$,

# EP 1 248 758 B1

the aprotic solvent is toluene and the concentration of Na tert.-amoxide is 41.3 to 53.2 wt.%.

9.  Solutions according to any one of claims 1 to 7, **characterised in that** M is Na and R is tert.-butyl $C(CH_3)_3$, the aprotic solvent is methyl tert.-butyl ether and the concentration of Na tert.-butoxide is 23.4 to 27.7 wt.%.

10. A process for the production of the alkoxide solutions containing alkali metal hydroxide stated in claims 1 to 9, **characterised in that** an anhydrous mixture of a secondary or tertiary alkali alkoxide ROM and alkali metal hydroxide M'OH is dissolved in an anhydrous, aprotic solvent.

11. A process for the production of the alkoxide solutions containing alkali metal hydroxide stated in claims 1 to 9, **characterised in that** a mixture of a secondary or tertiary alkali alkoxide ROM and alkali metal hydroxide M'OH is dissolved in an aprotic solvent and the solution is post-dried.

12. A process for the production of the alkoxide solutions containing alkali metal hydroxide stated in claims 1 to 9, **characterised in that** a mixture consisting of water and/or alkali metal hydroxide, a secondary or tertiary alcohol and an aprotic solvent is reacted with a metalation agent, wherein the metalation agent used is an alkali metal (Li, Na, K, Rb or Cs) or an alkali metal hydride or an alkali metal amide or an organoalkali metal compound.

13. A process according to claim 12, **characterised in that** butyllithium is used as the organoalkali metal compound.

14. Use of the alkoxide solutions stated in claims 1 to 9 as reagents for organic synthesis.


**Revendications**

1.  Solutions d'alcoolates alcalins ROM constitués d'un cation de métal alcalin et d'un reste d'alcool secondaire ou tertiaire, dans des solvants aprotiques, **caractérisées en ce qu'**elles contiennent au minimum 1,0 % en moles et au maximum 15 % en moles d'hydroxyde de métal alcalin M'OH et au maximum 1,0 % en moles d'alcool libre R-OH, ces pourcentages étant rapportés à l'alcoolate, R désignant un groupe alkyle secondaire ou tertiaire, ayant 3 à 20 atomes de carbone, et M et M' désignant, indépendamment l'un de l'autre, Li, Na, K, Rb ou Cs.

2.  Solutions selon la revendication 1, **caractérisées en ce que** la teneur en hydroxyde de métal alcalin M'OH atteint au minimum 1,5 % en moles.

3.  Solutions selon la revendication 1 ou 2, **caractérisées en ce que** la teneur en hydroxyde de métal alcalin M'OH atteint au maximum 12 % en moles.

4.  Solutions selon la revendication 1 ou 2, **caractérisées en ce que** la teneur en hydroxyde de métal alcalin M'OH atteint au maximum 10 % en moles.

5.  Solutions selon la revendication 1 ou 2, **caractérisées en ce que** la teneur en hydroxyde de métal alcalin M'OH atteint au maximum 8 % en moles.

6.  Solutions selon l'une quelconque des revendications 1 à 5, **caractérisées en ce qu'**elles contiennent au maximum 0,5 % en moles d'alcool libre R-OH.

7.  Solutions selon l'une quelconque des revendications 1 à 6, **caractérisées en ce qu'**on utilise comme solvant aprotique, un hydrocarbure (aromatique, ou aliphatique à chaîne ouverte ou cyclique), un éther (à chaîne ouverte ou cyclique, monofonctionnel ou plurifonctionnel), un amide, un acétal, un nitrile, ou un mélange de ces produits.

8.  Solutions selon l'une quelconque des revendications 1 à 7, **caractérisées en ce que** M représente Na, R représente le groupe tert-amyle $C(CH_3)_2C_2H_5$, le solvant aprotique est le toluène et la concentration du tert-amylate de sodium est de 41,3 à 53,2 % en poids.

9.  Solutions selon l'une quelconque des revendications 1 à 7, **caractérisées en ce que** M représente Na, R représente le groupe tert-butyle $C(CH_3)_3$, le solvant aprotique est l'oxyde de méthyle et de tert-butyle, et la concentration du tert-butylate de sodium est de 23,4 à 27,7 % en poids.

**10.** Procédé de préparation des solutions d'alcoolates, contenant un hydroxyde de métal alcalin, indiquées dans les revendications 1 à 9, **caractérisé en ce que** l'on dissout un mélange anhydre d'un alcoolate alcalin secondaire ou tertiaire ROM et d'un hydroxyde de métal alcalin M'OH dans un solvant aprotique anhydre.

**11.** Procédé de préparation des solutions d'alcoolates, contenant un hydroxyde de métal alcalin, indiquées dans les revendications 1 à 9, **caractérisé en ce que** l'on dissout un mélange d'un alcoolate alcalin secondaire ou tertiaire ROM et d'un hydroxyde de métal alcalin M'OH dans un solvant aprotique, et on sèche ensuite la sol ution.

**12.** Procédé de préparation des solutions d'alcoolates, contenant un hydroxyde de métal alcalin, indiquées dans les revendications 1 à 9, **caractérisé en ce que** l'on fait réagir un mélange constitué d'eau et/ou d'un hydroxyde de métal alcalin, d'un alcool secondaire ou tertiaire et d'un solvant aprotique, avec un agent de métallation qui est un métal alcalin (Li, Na, K, Rb ou Cs), un hydrure de métal alcalin, un amidure de métal alcalin, ou un dérivé organique d'un métal alcalin.

**13.** Procédé selon la revendication 12, **caractérisé en ce que** on utilise du butyllithium en tant que dérivé organique d'un métal alcalin.

**14.** Utilisation des solutions d'alcoolates indiquées dans les revendications 1 à 9, comme réactifs pour les synthèses organiques.

Fig. 1: Löslichkeit von NTA
in Toluol in Abhängigkeit vom NaOH-Gehalt
bei 0°C und RT